# EUROPEAN PATENT APPLICATION

(11) **EP 4 474 031 A1**
(43) Date of publication of application: **11.12.2024**
(21) Application number: 23749630.2
(22) Date of filing: 25.01.2023
(51) Int. Cl.: A63H 11/00, A63H 3/02, A63H 33/26, B25J 13/08

(54) **ROBOT**

(30) Priority: 02.02.2022 JP 2022014594
(71) Applicant: Nitto Denko Corporation, Ibaraki-shi, Osaka 567-8680 (JP)
(72) Inventor: FUKUSHIMA, Rihito, Ibaraki-shi, Osaka 567-8680 (JP); SHIMIZU, Yusuke, Ibaraki-shi, Osaka 567-8680 (JP); TOYODA, Yoshihiro, Ibaraki-shi, Osaka 567-8680 (JP); YAMAUCHI, Kengo, Ibaraki-shi, Osaka 567-8680 (JP)
(74) Representative: Hoffmann Eitle
(86) International application number: PCT/JP2023/002331
(87) International publication number: WO 2023/149314

(57) **Abstract**

Provided is a robot configured to naturally obtain biological information. The robot includes an external member; and an electromagnetic wave sensor that is provided internally of the external member and is configured to obtain biological information of a user by using electromagnetic waves.

## Description

### TECHNICAL FIELD

The present disclosure relates to robots.

### BACKGROUND ART

Robots including sensors and configured to collect users' biological information are known. The biological information collected by the robots is used, for example, in systems that support users' security, safety, or healthy life.

In order for users to continuously enjoy health supports without making the users conscious of diagnosis, a disclosed one of the robots as described above is configured to obtain users' biological information, in response to contact of the users with the robot, from a position of the contact of the users therewith, without requesting the users to contact the robot (see, for example, Patent Document 1).

### RELATED ART DOCUMENTS

### PATENT DOCUMENTS

Patent Document 1: Japanese Patent No. 6519560

### SUMMARY OF THE INVENTION

### PROBLEMS TO BE SOLVED BY THE INVENTION

In recent years, the number of single-person households is increasing owing to, for example, population decline and improvement in convenience of living in developed countries. As a result, the number of people who feel stress, such as loneliness or the like due to lack of communication, is on the increase. Therefore, there is an increasing demand for robots that can observe users' stress states based on biological information obtained from the users, relieve the users' stress through communication with the users, and provide healing to the users. However, the robot described in Patent Document 1 obtains biological information in response to users' contacts with the robot. Thus, the users are restrained upon obtainment of the biological information, and there is a concern that stress may sometimes be caused to the users. Therefore, there is a room for improvement from the viewpoint of naturally obtaining the biological information.

It is an object of the present invention to provide a robot configured to naturally obtain biological information.

### MEANS FOR SOLVING THE PROBLEMS

A robot according to an aspect of the present invention includes: an external member; and an electromagnetic wave sensor that is provided internally of the external member and is configured to obtain biological information of a user by using electromagnetic waves.

### ADVANTAGEOUS EFFECTS OF THE INVENTION

According to the present invention, it is possible to provide a robot configured to naturally obtain biological information.

### BRIEF DESCRIPTION OF THE DRAWINGS

[FIG. 1] FIG. 1 is a perspective view illustrating a robot according to an embodiment.
[FIG. 2] FIG. 2 is a side view of the robot of FIG. 1.
[FIG. 3] FIG. 3 is a cross-sectional view taken along a cut line III-III in FIG. 2.
[FIG. 4] FIG. 4 is a view illustrating a configuration of a vital sensor according to an embodiment.
[FIG. 5] FIG. 5 is a block diagram illustrating a hardware configuration of a controller according to an embodiment.
[FIG. 6] FIG. 6 is a block diagram illustrating a functional configuration of the controller according to the embodiment.
[FIG. 7] FIG. 7 is a flowchart illustrating a process of the controller according to the embodiment.

### DETAILED DESCRIPTION OF THE INVENTION

Hereinafter, embodiments of the invention will be described with reference to the drawings. In the drawings, the same components are denoted by the same reference symbols, and redundant description thereof will be omitted as appropriate.

The following embodiments illustrate a robot that embodies the technical idea of the present invention, and should not be construed as limiting the present invention thereto. Dimensions, materials, shapes, relative arrangements, and the like of the components described below are intended to illustrate the present invention, not limit the scope of the present invention thereto, unless otherwise specified. In addition, sizes, positional relationships, and the like of the members as illustrated in the drawings may be exaggerated in order to clarify the description.

### <Example of Overall Configuration of Robot 100>

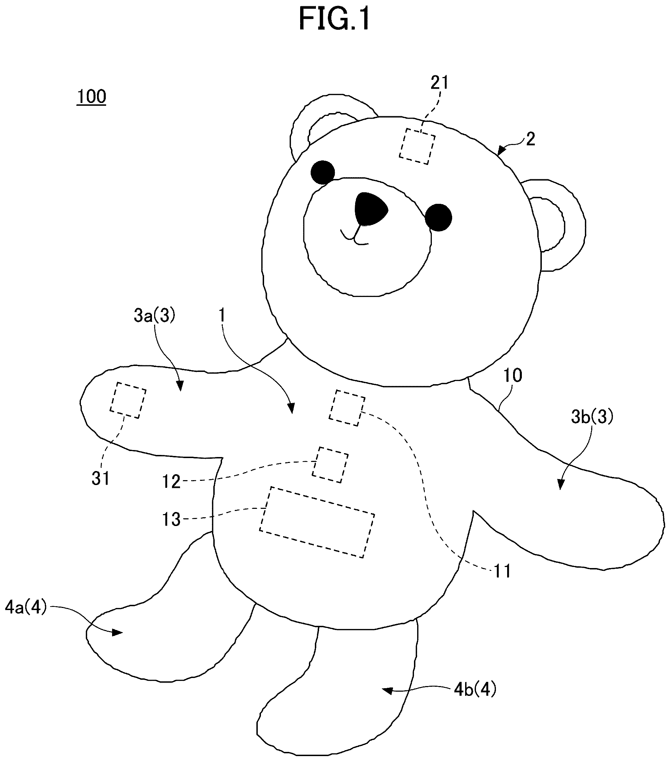

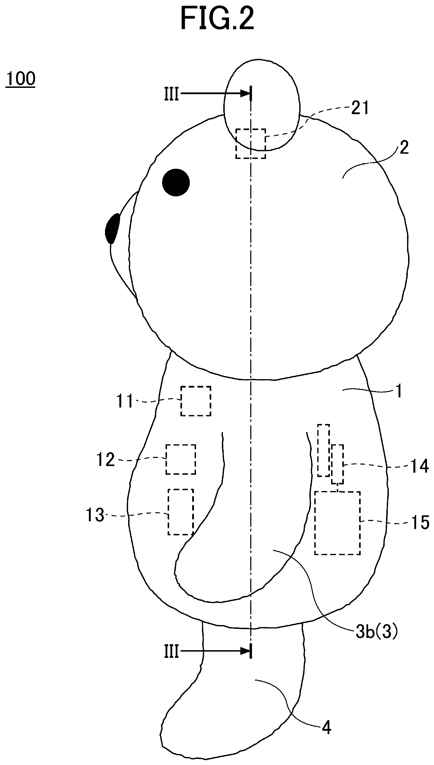

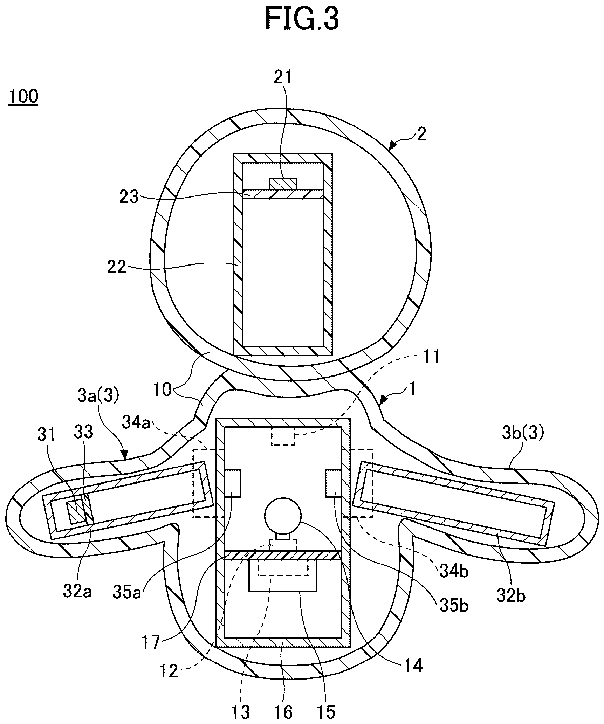

The configuration of the robot 100 according to an embodiment will be described with reference to FIGS. 1 to 3. FIG. 1 is a perspective view illustrating a robot 100 according to the embodiment. FIG. 2 is a side view of the robot 100. FIG. 3 is a cross-sectional view taken along the cut line III-III in FIG. 2

The robot 100 includes an external member 10 and is configured to be driven by supplied power. The robot 100 exemplified in the present embodiment is a doll-shaped robot that mimics a bear cub. The robot 100 is produced to have a size and weight suitable for being held by a user. Here, the user means a user of the robot 100. The robot 100 can observe the stress state of the user based on biological information obtained from the user, and can relieve the stress of the user through communication with the user and provide healing to the user. The communication in this specification includes: exchange or communication only in language; and exchange or communication involving contact or touch. For example, a state in which the user holds the robot 100 without uttering and communicates with the robot 100 while contacting the robot 100 is also included in the state in which the user and the robot 100 communicate with each other.

The external member 10 has softness. For example, the softness of the external member 10 is such that the hardness thereof is greater than Asker F0 and is Asker C70 or less. For example, the external member 10 includes a soft material that makes the user of the robot 100 feel comfortable upon touching the robot 100. The material of the external member 10 may be a material including an organic material, such as urethane foam, rubber, resin, fiber, or the like. The external member 10 may further include a soft cloth or the like that covers the outer surface of a base of a foam material or the like. Examples of the foam material include polystyrene, polyethylene, phenol, rubber-based foam materials, rubber-based sponge, and the like. Examples of the cloth include fabric, brushed fabric, leather, and the like. The foam material is readily processed through molding or the like. Thus, by using the foam material for the external member 10, mass productivity of the external member 10 can be increased.

The external member 10 may be formed of a cushioning material in which a fluid is enclosed by a resin film, such as polyethylene or the like. Examples of the cushioning material include: air cushions in which air is enclosed by a resin film; cushioning materials in which a liquid or gel is enclosed by a resin film; and the like.

As an example, the robot 100 includes a torso 1, a head 2, an arm 3, and a leg 4. The arm 3 includes a right arm 3a and a left arm 3b, and the leg 4 includes a right leg 4a and a left leg 4b. Here, the torso 1 corresponds to a robot body. The head 2, the arm 3, and the leg 4 each correspond to a driven body that is connected to the robot body so as to be displaceable relative to the robot body.

In the present embodiment, the arm 3 is configured to be displaceable relative to the torso 1. For example, when the robot 100 is held by the user, the right arm 3a and the left arm 3b are displaced to contact the user's neck, body, or the like so as to embrace the user. By this action, the user feels a sense of closeness to the robot 100. This promotes communication between the user and the robot 100.

The torso 1, the head 2, the arm 3, and the leg 4 are all covered by the external member 10. The external member of the torso 1 and the external member of the arm 3 are integrated. The external member of the head 2 is separated from the external members of the torso 1 and the arm 3. The leg 4 internally includes no components, such as a sensor and the like, and is formed only of the external member. However, this is by no means a limitation. For example, only a portion of the robot 100 that is more likely to be contacted by the user may be covered by the external member 10. At least one of the external members 10 of the torso 1, the head 2, the arm 3, and the leg 4 may be separated from the other external members. Alternatively, non-displaced portions of the head 2, the arm 3, and the leg 4 may internally include no components, such as a sensor and the like, and may be formed only of the external member 10.

The robot 100 includes a camera 11, a tactile sensor 12, a controller 13, a vital sensor 14, a battery 15, a first capacitive sensor 21, and a second capacitive sensor 31 internally of the external member 10.

More specifically, as illustrated in FIG. 3, the robot 100 includes a torso frame 16 and a torso stage 17 internally of the external member 10 of the torso 1. Also, the robot 100 includes a head frame 22 and a head stage 23 internally of the external member 10 of the head 2. Further, the robot 100 includes a right arm frame 32a and a right arm stage 33 internally of the external member 10 of the right arm 3a, and the robot 100 includes a left arm frame 32b internally of the external member 10 of the left arm 3b.

The torso frame 16, the head frame 22, the right arm frame 32a, and the left arm frame 32b are structures formed by combining a plurality of columnar members. The torso stage 17, the head stage 23, and the right arm stage 33 are plate-like members having stage surfaces. The torso stage 17 is fixed to the torso frame 16, the head stage 23 is fixed to the head frame 22, and the right arm stage 33 is fixed to the right arm frame 32a. The torso frame 16, the head frame 22, the right arm frame 32a, and the left arm frame 32b may be formed in a box shape including a plurality of plate-like members.

The right arm frame 32a is connected to the torso frame 16 via a right arm connecting mechanism 34a, and is displaceable relative to the torso frame 16 by being driven by a right arm servo motor 35a. In response to displacement of the right arm frame 32a, the right arm 3a is displaced relative to the torso 1.

The left arm frame 32b is connected to the torso frame 16 via a left arm connecting mechanism 34b, and is displaceable relative to the torso frame 16 by being driven by a left arm servo motor 35b. In response to displacement of the left arm frame 32b, the left arm 3b is displaced relative to the torso 1.

The camera 11 is fixed to the torso frame 16. The tactile sensor 12, the controller 13, the vital sensor 14, and the battery 15 are fixed to the torso stage 17. The controller 13 and the battery 15 are fixed to the torso stage 17 on a side thereof opposite to the side on which the tactile sensor 12 and the vital sensor 14 are fixed. Here, the controller 13 and the battery 15 are arranged like this in consideration of a space available for arrangement thereof on the torso stage 17, and the above arrangement is not necessarily limiting. However, when the battery 15 is fixed to the torso stage 17 on the side thereof opposite to the side on which the tactile sensor 12 and the vital sensor 14 are fixed, the center of gravity of the robot 100 becomes lower because the battery 15 is heavier than the other components. When the center of gravity of the robot 100 is low, the position, posture, or both of the robot 100 becomes more stable, and charging, replacement, or both of the battery 15 is readily performed, which is preferable.

The first capacitive sensor 21 is fixed to the head stage 23, and the second capacitive sensor 31 is fixed to the right arm stage 33. The fixing of the camera 11, the tactile sensor 12, the controller 13, the vital sensor 14, the battery 15, the first capacitive sensor 21, the second capacitive sensor 31, and the like can be performed by means of a screw, an adhesive, and the like.

No particular limitation is imposed on the materials of the torso frame 16, the torso stage 17, the head frame 22, the head stage 23, the right arm frame 32a, the right arm stage 33, and the left arm frame 32b. The materials thereof can be resin, metal, and the like. However, from the viewpoint of securing strength during driving, it is preferable to use metal, such as aluminum or the like, for the torso frame 16, the right arm frame 32a, and the left arm frame 32b. Meanwhile, if it is possible to secure strength, it is preferable to use resin as the materials of these parts in order to reduce the weight of the robot 100. No particular limitation is imposed on the materials of the torso stage 17, the head frame 22, the head stage 23, the right arm stage 33, and the left arm frame 32b. The materials thereof can be resin or metal. However, from the viewpoint of reducing the weight of the robot 100, it is preferable to use resin.

The controller 13 is communicably connected to the camera 11, the tactile sensor 12, the vital sensor 14, the first capacitive sensor 21, the second capacitive sensor 31, the right arm servo motor 35a, and the left arm servo motor 35b in a wired or wireless manner.

The camera 11 is an image sensor configured to output a captured image of the surroundings of the robot 100 to the controller 13. In the present embodiment, the camera 11 is an example of a capturing unit configured to capture an image of a user. The camera 11 includes a lens, and a capturing element configured to capture an image formed by the lens. The capturing element may be a CCD (Charge Coupled Device), a CMOS (Complementary Metal-Oxide Semiconductor), or the like. The captured image may be either a still image or a moving image.

The tactile sensor 12 is a sensor element configured to detect information to be felt by the tactile sense of the human hand or the like, convert the detected information to a tactile signal that is an electric signal, and output the tactile signal to the controller 13. For example, the tactile sensor 12 converts information on pressure and vibration, generated by the user's contact with the robot 100, to a tactile signal by a piezoelectric element, and outputs the tactile signal to the controller 13.

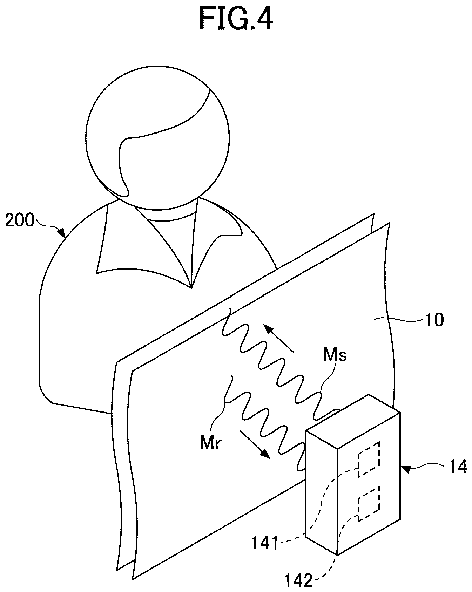

The vital sensor 14 is an example of the electromagnetic wave sensor configured to obtain the biological information of the user by using the electromagnetic waves. The vital sensor 14 will be described below in detail with reference to FIG. 4.

The first capacitive sensor 21 and the second capacitive sensor 31 are sensor elements configured to output a capacitance signal to the controller 13, the capacitance signal being obtained by detecting user's contact or proximity to the robot 100 in accordance with a change in capacitance. The first capacitive sensor 21 is preferably a rigid sensor having no flexibility from the viewpoint of stabilizing the external member 10. The arm 3 is a portion that is likely to be touched by the user. Thus, the second capacitive sensor 31 is preferably a flexible sensor including a conductive thread or the like from the viewpoint of improving texture.

All of the tactile sensor 12, the first capacitive sensor 21, and the second capacitive sensor 31 correspond to a user detector configured to detect the user's contact or proximity to the robot 100.

The battery 15 is a power supply configured to supply power to the camera 11, the tactile sensor 12, the controller 13, the vital sensor 14, the first capacitive sensor 21, the second capacitive sensor 31, the right arm servo motor 35a, and the left arm servo motor 35b. Various types of secondary batteries, such as lithium ion batteries, lithium polymer batteries, and the like, can be used as the battery 15.

The camera 11, the tactile sensor 12, the first capacitive sensor 21, and the second capacitive sensor 31 in the robot 100 are not essential components. The robot 100 only needs to include at least the vital sensor 14 internally of the external member 10. Positions at which these components are to be disposed can be changed as appropriate.

The robot 100 does not necessarily need to include the controller 13 internally of the external member 10. The controller 13 can communicate with the components via radio waves from the exterior of the external member 10. The battery 15 can also supply power to the components from the exterior of the external member 10.

The arm 3 may be an articulated robot arm including a plurality of frame members and a plurality of connecting mechanisms. When the arm 3 is formed by an articulated robot arm, the robot 100 can achieve a more realistic embracing motion.

In the present embodiment, a configuration in which only the arm 3 is displaceable is exemplified. However, the present invention is not limited to this. The head 2, the arm 3, the leg 4, or any combination thereof may be displaceable. The configuration and shape of the robot 100 are not limited to those exemplified in the present embodiment, and can be appropriately changed in accordance with a user's preference, a usage mode of the robot 100, and the like.

### <Configuration Example of Vital Sensor 14>

FIG. 4 is a view illustrating a configuration of the vital sensor 14. The vital sensor 14 is a microwave Doppler sensor that includes a microwave emitter 141 and a microwave receiver 142. Microwaves are an example of electromagnetic waves.

The microwave emitter 141 of the vital sensor 14 is configured to emit emitted waves Ms, i.e., microwaves, toward a user 200 from the interior of the external member 10 in the robot 100. The microwave receiver 142 of the vital sensor 14 is configured to receive reflected waves Mr of the emitted waves Ms reflected by the user 200.

The vital sensor 14 uses the Doppler effect from the difference between the frequency of the emitted waves Ms and the frequency of the reflected waves Mr, thereby detecting, in a non-contact manner, minute displacement generated on the body surface due to, for example, the beating of the heart of the user 200. The non-contact as used herein means that the vital sensor 14 does not contact or does not continue to contact the body surface of the user 200. Also, when the vital sensor 14 detects minute displacement generated on the body surface of the user 200 through the clothes, gloves, or the like worn by the user 200, the above non-contact means that the vital sensor 14 does not contact or does not continue to contact the clothes, gloves, or the like worn by the user 200. The vital sensor 14 can detect minute displacement generated on the body surface of the user 200 even in a state in which the user 200 is in contact with the robot 100, such as, for example, a state in which the user 200 is holding the robot 100. The vital sensor 14 can obtain information, such as heartbeat, respiration, pulse waves, blood pressure, and the like, as biological information of the user 200 from the detected minute displacement, and output the obtained biological information to the controller 13.

The vital sensor 14 is not limited to a microwave Doppler sensor. The vital sensor 14 may be a sensor configured to detect minute displacement generated on the body surface by using a change in coupling between the human body and an antenna, or may be a sensor configured to utilize electromagnetic waves other than microwaves, such as near-infrared light or the like. The vital sensor 14 may be a millimeter wave radar, a microwave radar, or the like.

Here, electromagnetic waves having a high frequency, such as infrared light, visible light, and the like, have a low transmittance with respect to the external member 10. Thus, the signal intensity of the electromagnetic waves detected by the vital sensor 14 becomes lower, and the detection accuracy of minute displacement generated on the body surface of the user 200 may be low. Also, it may be challenging for electromagnetic waves having a high frequency, such as infrared light, visible light, and the like, to detect a signal of biological information having a long displacement period, such as heartbeat, respiration, and the like. Meanwhile, for electromagnetic waves having a low frequency, the detection resolution of minute displacement generated on the body surface of the user 200 may become lower.

From the viewpoint of increasing the detection accuracy of minute displacement generated on the body surface of the user 200, enabling detection of a signal of biological information having a long displacement period, and increasing the detection resolution of minute displacement generated on the body surface of the user 200, the frequency of electromagnetic waves in the vital sensor 14 is preferably 30 MHz or higher and 100 THz or lower. From the above-described viewpoint, the frequency of electromagnetic waves in the vital sensor 14 is more preferably 300 MHz or higher and 300 GHz or lower, and especially preferably 1 GHz or higher and 100 GHz or lower. Also, from the above-described viewpoint, the thickness of the external member 10 is preferably 0.1 mm or larger and 1,000 mm or smaller, more preferably 1 mm or larger and 500 mm or smaller, and especially preferably 5 mm or larger and 200 mm or smaller. The above conditions in relation to the frequency of the electromagnetic waves and the above conditions in relation to the thickness of the external member 10 may be combined together. The thickness of the external member 10 in the present specification means the thickness thereof in a state in which the robot 100 is being used.

In the present embodiment, the external member 10 may have a transmittance of 1% or higher with respect to electromagnetic waves having a frequency of 300 MHz or higher and 300 GHz or lower. When the transmittance of the electromagnetic waves having the frequency of 300 MHz or higher and 300 GHz or lower is 1% or higher, the thickness of the external member 10 may be 0.1 mm or larger and 1,000 mm or smaller. By satisfying these conditions, in the present embodiment, it is possible to increase the detection accuracy of minute displacement generated on the body surface of the user 200, detect a signal of biological information having a long displacement period, and increase the detection resolution of minute displacement generated on the body surface of the user 200.

In the present embodiment, the biological information detected by the vital sensor 14 may include pulse, blood pressure, heartbeat, respiration, or any combination thereof. More specifically, the biological information detected by the vital sensor 14 may include heartbeat interval, heartbeat variation, LF, HF, LF/HF, pulse wave, pulse, pulse interval R-R, pulse waveform, pulse wave velocity, hemodynamics, pulse pressure, body temperature, brain wave, blood sugar, respiration, respiratory rate, rhythm, depth of respiration, and the like. By obtaining and analyzing these types of biological information, in the present embodiment, it is possible to obtain information in relation to the psychological or health state of the user 200.

In the present embodiment, the vital sensor 14 is provided internally of the external member 10, and thus the user 200 cannot visually recognize the vital sensor 14. This suppresses sensation of the user 200 to resist detection of his or her biological information, and as a result the biological information can be smoothly obtained. Also, the vital sensor 14 can obtain the biological information in a non-contact manner. Therefore, even if the user moves to some extent, the biological information can be obtained unlike in a contact-type sensor that requires the user to contact the same site thereof for a certain period.

Further, by promoting communication between the user 200 and the robot 100 by the embracing motion and the like of the robot 100, the robot 100 can obtain the biological information while being held by the user 200, i.e., in a state of being in contact with or proximity to the user 200. Thereby, the robot 100 can obtain the biological information with reduced noise and high reliability.

### <Example of Configuration of Controller 13>

### (Hardware Configuration Example)

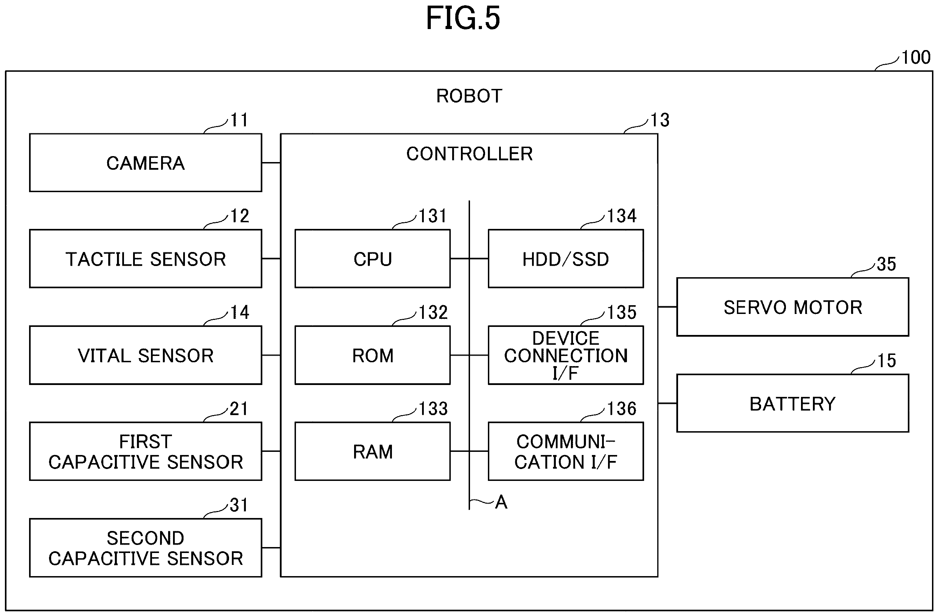

FIG. 5 is a block diagram illustrating the hardware configuration of the controller 13. The controller 13 is constructed by a computer, and includes a CPU (Central Processing Unit) 131, a ROM (Read Only Memory) 132, a RAM (Random Access Memory) 133, an HDD/SSD (Hard Disk Drive/Solid State Drive) 134, a device connection interface (I/F) 135, and a communication interface (I/F) 136. These are communicably connected to each other via a system bus A.

The CPU 131 is configured to execute control processes including various types of arithmetic processing. The ROM 132 is configured to store programs used to drive the CPU 131, such as IPL (Initial Program Loader) or the like. The RAM 133 is used as a work area of the CPU 131. The HDD/SSD 134 is configured to store various types of information, such as programs, biological information obtained by the vital sensor 14, information detected by various sensors, and the like.

The device connection I/F 135 is an interface configured to connect the controller 13 to various external devices. The external devices are the camera 11, the tactile sensor 12, the vital sensor 14, the first capacitive sensor 21, the second capacitive sensor 31, a servo motor 35, the battery 15, and the like. The servo motor 35 is a generic term of the right arm servo motor 35a and the left arm servo motor 35b.

The communication I/F 136 is an interface configured to communicate with an external device via a communication network or the like. For example, the controller 13 connects via the communication I/F 136 to the Internet, and communicates with the external device via the Internet.

At least some of the functions achieved by the CPU 131 may be achieved by an electric circuit or an electronic circuit.

### (Functional Configuration Example)

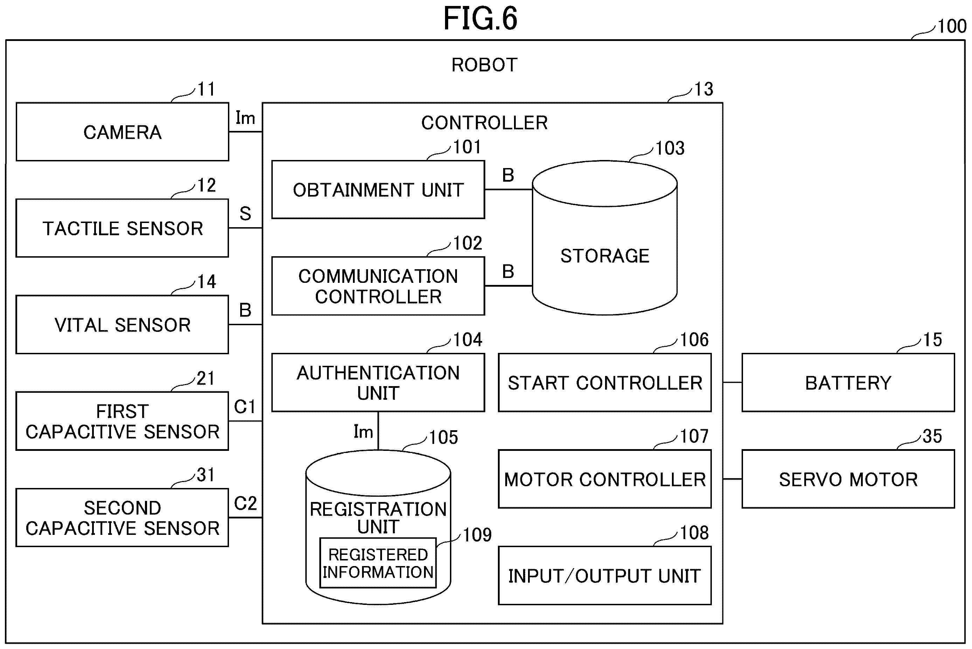

FIG. 6 is a block diagram illustrating a functional configuration of the controller 13. The controller 13 includes an obtainment unit 101, a communication controller 102, a storage 103, an authentication unit 104, a registration unit 105, a start controller 106, a motor controller 107, and an input/output unit 108.

The device connection I/F 135 or the like of the controller 13 can achieve the functions of the obtainment unit 101 and the input/output unit 108. The communication I/F 136 or the like of the controller 13 can achieve the functions of the communication controller 102. Also, the HDD/SSD 134 or the like of the controller 13 can achieve the functions of the storage 103 and the registration unit 105. The CPU 131 of the controller 13 can achieve the functions of the authentication unit 104, the start controller 106, and the motor controller 107 by executing the processes of the programs stored in the ROM 132 and the like. Some of the above functions of the controller 13 may be achieved by an external device, such as a PC (Personal Computer) or the like, or through distributed processing between the controller 13 and an external device.

The obtainment unit 101 is configured to obtain biological information B from the vital sensor 14 by controlling communication between the controller 13 and the vital sensor 14.

The communication controller 102 is configured to control communication with an external device via a communication network or the like. For example, the communication controller 102 can transmit the biological information B, obtained by the vital sensor 14, to the external device via the communication network.

The storage 103 is configured to store the biological information B obtained by the vital sensor 14. While the obtainment unit 101 obtains the biological information B from the vital sensor 14, the storage 103 continuously stores the obtained biological information B. The storage 103 can also store information obtained based on, for example, an image Im captured by the camera 11, a tactile signal S from the tactile sensor 12, a first capacitance signal C1 from the first capacitive sensor 21, and a second capacitance signal C2 from the second capacitive sensor 31.

The authentication unit 104 is configured to perform personal authentication of the user 200 based on the image Im of the user 200 captured by the camera 11. For example, the authentication unit 104 performs face authentication based on the image Im including the face of the user 200 captured by the camera 11, by referring to registered information 109 of the face image registered in advance in the registration unit 105. Thereby, it is possible to associate the user 200 that is currently in contact with or proximity to the robot 100, with the personal information registered in advance, and can associate the biological information B, obtained by the vital sensor 14, with the personal information. Also, the controller 13 can perform control so as to stop the vital sensor 14 from starting obtainment of the biological information when the face image included in the captured image Im is not registered in the registration unit 105.

The start controller 106 causes the vital sensor 14 to start obtainment of the biological information B based on the tactile signal S, the first capacitance signal C1, and the second capacitance signal C2 that are the detection results. For example, when the contact or proximity of the user 200 to the robot 100 is detected based on the detection results, the start controller 106 causes the vital sensor 14 to start obtainment of the biological information B by turning on a switch or the like configured to supply power from the battery 15 to the vital sensor 14.

The motor controller 107 controls driving of the servo motor 35.

The input/output unit 108 is configured to control communication between the controller 13 and an external device. For example, the input/output unit 108 can input the captured image Im, the tactile signal S, the first capacitance signal C1, the second capacitance signal C2, and the like, or can output information or signals to the external device.

### <Example of Processing by Controller 13>

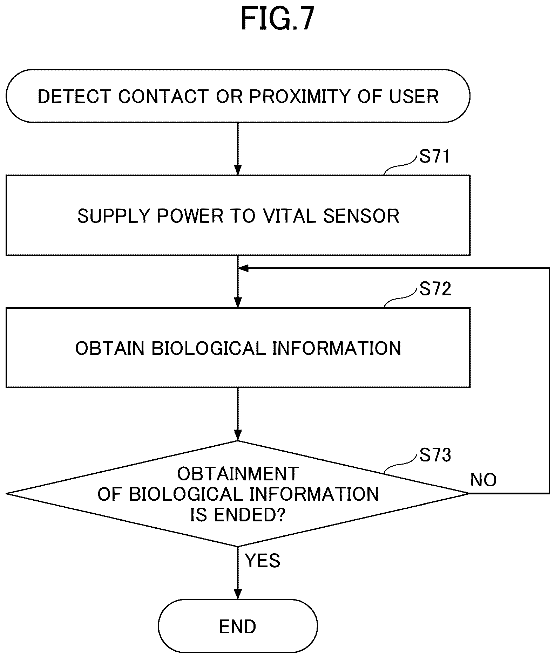

FIG. 7 is a flowchart illustrating a process of the controller 13. FIG. 7 illustrates the process through which the controller 13 causes the vital sensor 14 to obtain biological information. The controller 13 starts the process of FIG. 7 in response to detecting the contact or proximity of the user 200 to the robot 100 based on the tactile signal S, the first capacitance signal C1, the second capacitance signal C2, or any combination thereof. At the time of start, power is supplied from the battery 15 to the camera 11, the tactile sensor 12, the first capacitive sensor 21, the second capacitive sensor 31, and the servo motor 35, while no power is supplied to the vital sensor 14.

First, in step S71, the start controller 106 of the controller 13 causes the vital sensor 14 to start obtainment of the biological information B by turning on a switch or the like configured to supply power from the battery 15 to the vital sensor 14.

Subsequently, in step S72, the obtainment unit 101 of the controller 13 obtains the biological information B from the vital sensor 14 and stores the biological information B in the storage 103.

Subsequently, in step S73, when the controller 13 determines whether or not to end the obtainment of the biological information. For example, when the controller 13 determines to end the obtainment of the biological information when the contact or proximity of the user 200 to the robot 100 is no longer detected based on the tactile signal S, the first capacitance signal C1, the second capacitance signal C2, or any combination thereof. Alternatively, the controller 13 may determine to end the obtainment of the biological information when the quantity or period in which the biological information B is continuously obtained exceeds a predetermined threshold.

In step S73, when it is determined that the obtainment of the biological information is ended (step S73, Yes), the controller 13 ends the process, whereas when it is determined that the obtainment of the biological information is not ended (step S73, No), the controller 13 performs the process after step S72 again.

In this manner, the controller 13 can perform the obtainment process of the biological information B. At the start of the process as illustrated in FIG. 7, the vital sensor 14 may be in a standby state (sleep state) in which the quantity of power supplied is suppressed. That is, the controller 13 may cause the vital sensor 14 to start obtainment of the biological information B by restoring the vital sensor 14 from the standby state, in which the power supplied is suppressed, to a state in which the vital sensor 14 can obtain the biological information B.

### <Actions and Effects of Robot 100>

As described above, the robot 100 includes the external member 10, and the vital sensor 14 (electromagnetic wave sensor) configured to obtain the biological information B of the user 200 by using microwaves (electromagnetic waves). The vital sensor 14 is provided internally of the external member 10. By providing the vital sensor 14 internally of the external member 10, the vital sensor 14 cannot be visually recognized by the user 200. The vital sensor 14 can obtain the biological information B in a non-contact manner by using microwaves. Further, even if the user moves to some extent, the vital sensor 14 can obtain the biological information B unlike in a contact-type sensor that requires the user to contact the same site thereof for a certain period. This suppresses sensation of the user 200 to resist detection of his or her biological information, and as a result it is possible to provide the robot 100 configured to naturally obtain biological information B.

In the present embodiment, the external member 10 includes an organic material. Microwaves transmit through the organic material, and thus the vital sensor 14 provided internally of the external member 10 can obtain the biological information B.

In the present embodiment, the external member 10 has softness. For example, the external member 10 may include a soft portion that is a portion having a hardness that is greater than Asker F0 and Asker C70 or less. When the external member 10 has softness or the soft portion, the user 200 is more likely to touch the robot 100. Thereby, in the present embodiment, the communication between the user 200 and the robot 100 can be promoted.

In the present embodiment, the external member 10 may include a soft portion externally of the vital sensor 14. For example, the external member 10 includes a soft portion between: the vital sensor 14 disposed internally of the external member 10; and the user 200 in contact with the robot 100. When the external member 10 includes a soft portion externally of the vital sensor 14, the user 200 in contact with the robot 100 can feel softness of the soft portion without feeling the hardness of the vital sensor 14. Thereby, the user 200 is more likely to touch the robot 100, and thus the communication between the user 200 and the robot 100 can be promoted.

Further, in the present embodiment, the robot body may include a soft portion. When the robot body includes a soft portion, the user 200 is more likely to touch the robot 100. Thereby, in the present embodiment, the communication between the user 200 and the robot 100 can be promoted.

In the present embodiment, the robot body includes at least the torso 1, and the torso 1 may include a soft portion. The torso 1 includes the abdomen, chest, back, waist, buttocks, and the like of the robot 100. The torso 1 is a portion of the robot 100 that is contacted by the user 200 with high frequency. When the torso 1 includes a soft portion, the user 200 in contact with the robot 100 feels softness more frequently. Thereby, the user 200 is more likely to touch the robot 100, and in the present embodiment, the communication between the user 200 and the robot 100 can be promoted.

Also, in the present embodiment, the torso 1 may include at least the abdomen, and the abdomen may include a soft portion. The abdomen is a portion of the torso 1 that is contacted by the user 200 with especially high frequency. Thus, when the abdomen includes a soft portion, the user 200 feels softness with especially high frequency. Thereby, the user 200 is more likely to touch the robot 100, and in the present embodiment, the communication between the user 200 and the robot 100 can be promoted.

Also, in the present embodiment, the robot 100 includes the torso 1 (robot body) and the arm 3 (driven body) connected to the torso 1 so as to be displaceable relative to the torso 1. The robot 100 contacts the user 200 via the arm 3 and can perform, for example, an embracing motion of contacting the arm 3 with the neck, torso, or the like of the user 200. Thereby, the user 200 can feel a sense of closeness to the robot 100, and thus the communication between the user 200 and the robot 100 can be promoted.

Also, in the present embodiment, the robot 100 includes the camera 11 (capturing unit) configured to capture the image Im of the user 200, and the authentication unit 104 configured to perform personal authentication of the user 200 based on the image Im captured by the camera 11. The robot 100 can obtain the biological information B associated with the personal information by using the authentication result obtained by the authentication unit 104.

Also, in the present embodiment, the robot 100 includes the tactile sensor 12, the first capacitive sensor 21, and the second capacitive sensor 31 (user detectors) configured to detect contact or proximity of the user 200 to the robot 100. Also, the robot 100 includes the start controller 106 configured to cause the vital sensor 14 to start obtainment of the biological information B based on the tactile signal S, the first capacitance signal C1, the second capacitance signal C2, or any combination thereof (detection results). With this configuration, the biological information B is not obtained when the user 200 is away from the robot 100, and thus the robot 100 can suppress power to be consumed for obtainment of the biological information B.

Although preferable embodiments have been described above in detail, the present invention is not limited to the above-described embodiments. Various modifications and substitutions may be made to the above-described embodiments without departing from the scope of the claims recited.

Also, the numbers used in the above-described embodiments, such as ordinal numerals, quantities, and the like, are all exemplified to specifically describe the technique of the present invention, and the present invention is not limited to the exemplified numbers. The connection relationships between the components are exemplified to specifically describe the technique of the present invention, and the connection relationships that achieve the functions of the present invention are not limited thereto.

The robot according to the embodiments is especially suitable for applications for supporting the users' security, safety, or healthy life; however is not limited to these applications. The robot according to the embodiments can be used for obtaining biological information of various users.

Aspects of the present invention are, for example, as follows.
<1> A robot, including: an external member; and an electromagnetic wave sensor that is provided internally of the external member and is configured to obtain biological information of a user by using electromagnetic waves.
<2> The robot according to <1>, in which the external member includes an organic material.
<3> The robot according to <1> or <2>, in which the external member has softness.
<4> The robot according to <3>, in which the external member includes a soft portion, the soft portion being a portion having a hardness that is greater than Asker F0 and Asker C70 or less.
<5> The robot according to <4>, in which the external member includes the soft portion externally of the electromagnetic wave sensor.
<6> The robot according to <4> or <5>, in which a robot body includes the soft portion.
<7> The robot according to <6>, in which the robot body includes at least a torso, and the torso includes the soft portion.
<8> The robot according to <7>, in which the torso includes at least an abdomen, and the abdomen includes the soft portion.
<9> The robot according to any one of <1> to <8>, in which the external member has a transmittance of 1% or higher with respect to electromagnetic waves having a frequency of 300 MHz or higher and 300 GHz or lower.
<10> The robot according to <9>, in which a thickness of the external member is 0.1 mm or larger and 1,000 mm or smaller.
<11> The robot according to any one of <1> to <10>, further including: a robot body; and a driven body connected to the robot body so as to be displaceable relative to the robot body.
<12> The robot according to <11>, in which the driven body includes an arm that is displaceable relative to the robot body.
<13> The robot according to any one of <1> to <12>, further including: a capturing unit configured to capture an image of the user; and an authentication unit configured to perform personal authentication of the user based on the image captured by the capturing unit.
<14> The robot according to any one of <1> to <12>, further including: a user detector configured to detect contact or proximity of the user to the robot; and a controller configured to cause the electromagnetic wave sensor to start obtainment of the biological information based on a detection result obtained by the user detector.

This application claims priority to Japanese Patent Application No. 2022-014594 filed with the Japan Patent Office on February 2, 2022, and the entire contents of this Japanese patent application are incorporated herein by reference.

### REFERENCE SIGNS LIST

- 1: Torso
- 2: Head
- 3: Arm
- 3a: Right arm
- 3b: Left arm
- 4: Leg
- 4a: Right leg
- 4b: Left leg
- 10: External member
- 11: Camera
- 12: Tactile sensor
- 13: Controller
- 14: Vital sensor (electromagnetic wave sensor)
- 141: Microwave emitter
- 142: Microwave receiver
- 15: Battery
- 16: Torso frame
- 17: Torso stage
- 21: First capacitive sensor
- 22: Head frame
- 23: Head stage
- 31: Second capacitive sensor
- 32a: Right arm frame
- 32b: Left arm frame
- 33: Right arm stage
- 34a: Right arm connecting mechanism
- 34b: Left arm connecting mechanism
- 35: Servo motor
- 35a: Right arm servo motor
- 35b: Left arm servo motor
- 100: Robot
- 101: Obtainment unit
- 102: Communication controller
- 103: Storage
- 104: Authentication unit
- 105: Registration unit
- 106: Start controller
- 107: Motor controller
- 108: Input/output unit
- 109: Registered information
- 131: CPU
- 132: ROM
- 133: RAM
- 134: HDD/SSD
- 135: Device connection I/F
- 136: Communication I/F
- 200: User
- A: System bus
- B: Biological information
- C1: First capacitance signal
- C2: Second capacitance signal
- Im: Captured image
- Ms: Emitted waves
- Mr: Reflected waves

## Claims

1. A robot, comprising:
an external member; and
an electromagnetic wave sensor that is provided internally of the external member and is configured to obtain biological information of a user by using electromagnetic waves.

2. The robot according to claim 1, wherein
the external member includes an organic material.

3. The robot according to claim 1, wherein
the external member has softness.

4. The robot according to claim 3, wherein
the external member includes a soft portion, the soft portion being a portion having a hardness that is greater than Asker F0 and Asker C70 or less.

5. The robot according to claim 4, wherein
the external member includes the soft portion externally of the electromagnetic wave sensor.

6. The robot according to claim 4, wherein
a robot body includes the soft portion.

7. The robot according to claim 6, wherein
the robot body includes at least a torso, and
the torso includes the soft portion.

8. The robot according to claim 7, wherein
the torso includes at least an abdomen, and
the abdomen includes the soft portion.

9. The robot according to claim 3, wherein
the external member has a transmittance of 1% or higher with respect to electromagnetic waves having a frequency of 300 MHz or higher and 300 GHz or lower.

10. The robot according to claim 9, wherein
a thickness of the external member is 0.1 mm or larger and 1,000 mm or smaller.

11. The robot according to claim 3, further comprising:
a robot body; and
a driven body connected to the robot body so as to be displaceable relative to the robot body.

12. The robot according to claim 11, wherein
the driven body includes an arm that is displaceable relative to the robot body.

13. The robot according to any one of claims 1 to 12, further comprising:
a capturing unit configured to capture an image of the user; and
an authentication unit configured to perform personal authentication of the user based on the image captured by the capturing unit.

14. The robot according to any one of claims 1 to 12, further comprising:
a user detector configured to detect contact or proximity of the user to the robot; and
a controller configured to cause the electromagnetic wave sensor to start obtainment of the biological information based on a detection result obtained by the user detector.
